# EUROPEAN PATENT SPECIFICATION

(11) **EP 3 395 830 B1**
(45) Date of publication and mention of the grant of the patent: **14.12.2022**
(21) Application number: 16878635.8
(22) Date of filing: 19.12.2016
(51) Int. Cl.: A61K 9/16, A61K 9/51, C07K 14/78, A61K 9/14, A61K 47/42, A61K 49/00, A61K 49/04, A61K 51/00, C08J 3/12, C08J 3/24, C12N 5/071

(54) **GELATIN PARTICLES, METHOD FOR PRODUCING GELATIN PARTICLES, GELATIN PARTICLE-ENCAPSULATING CELLS, AND METHOD FOR PRODUCING GELATIN PARTICLE-ENCAPSULATING CELLS**
GELATINEPARTIKEL, VERFAHREN ZUR HERSTELLUNG VON GELATINEPARTIKELN, GELATINEPARTIKELVERKAPSELUNGSZELLE UND VERFAHREN ZUR HERSTELLUNG VON GELATINEPARTIKELVERKAPSELUNGSZELLEN
PARTICULES DE GÉLATINE, PROCÉDÉ DE PRODUCTION DE PARTICULES DE GÉLATINE, CELLULES ENCAPSULANT DES PARTICULES DE GÉLATINE, ET PROCÉDÉ DE PRODUCTION DE CELLULES ENCAPSULANT DES PARTICULES DE GÉLATINE

(30) Priority: 25.12.2015 JP 2015254954; 23.06.2016 JP 2016124507
(43) Date of publication of application: 31.10.2018
(73) Proprietor: Konica Minolta, Inc., Tokyo 100-7015 (JP); Tabata, Yasuhiko, Uji-shi Kyoto 611-0024 (JP)
(72) Inventor: TABATA, Yasuhiko, Uji-city Kyoto 611-0024 (JP); HIRAYAMA, Natsumi, Tokyo 100-7015 (JP); INUI, Chie, Tokyo 100-7015 (JP); MAEZAWA, Akihiro, Tokyo 100-7015 (JP)
(74) Representative: Neilson, Martin Mark
(86) International application number: PCT/JP2016/087809
(87) International publication number: WO 2017/110746

(56) References cited:
- WO-A1-2008/062908
- WO-A1-2014/188888
- WO-A1-2014/192909
- JP-A- 2008 150 596
- JP-A- 2009 519 894
- DOI, N. ET AL.: 'Preparation of Biodegradable Gelatin Nanospheres with a Narrow Size Distribution for Carrier of Cellular Internalization of Plasmid DNA, 2012' JOURNAL OF BIOMATERIALS SCIENCE vol. 23, no. 8, January 2012, pages 991 - 1004, XP055495396
- ISHIKAWA, H. ET AL.: 'Gelatin nanospheres incorporating siRNA for controlled intracellular release' BIOMATERIALS vol. 33, no. 35, December 2012, pages 9097 - 9104, XP055393420
- Anonymous: "Horiba Scientific, A Guidebook to Particle Size Analysis", , 1 January 2012 (2012-01-01), pages 1-32, XP055060732, Irvine, CA 92618, USA Retrieved from the Internet: URL:http://www.horiba.com/fileadmin/upload s/Scientific/eMag/PSA/Guidebook/pdf/PSA_Gu idebook.pdf [retrieved on 2013-04-23]

## Description

### Technical Field

The present invention relates to a method for producing gelatin particles, and a method for producing a gelatin particle-containing cell.

### Background Art

Gelatin is a material that is highly biocompatible and is degraded and readily absorbed by the body. Therefore, gelatin is sometimes used to produce capsules to encapsulate foods or drugs.

For example, Patent Literature 1 discloses swellable gelatin particles made of thermally-crosslinked gelatin having a jelly strength of 80 to 120 g. The dried gelatin particles before swelling have a particle diameter of 20 to 1600 µm, and the dried gelatin particles after swelling have a particle diameter of 50 to 2000 µm. According to Patent Literature 1, the swellable gelatin particles have excellent shape retentivity and are hard to break even when deformed by the application of external stress, and are therefore suitable for intravascular administration using a microcatheter or a syringe needle. Patent Literature 1 describes that the time required for complete dissolution of the gelatin particles can be controlled by thermally self-crosslinking gelatin constituting the gelatin particles.

Further, Patent Literature 2 discloses gelatin particles that consist essentially of an aqueous gelatin gel and have an average diameter of 350 nm or less and a narrow size distribution. According to Patent Literature 2, the gelatin nanoparticles can selectively deliver and release a carried active substance, and are therefore suitable for targeted delivery of an active substance in the body. Patent Literature 2 describes that the degradation behavior of the gelatin particles can be controlled by chemically crosslinking gelatin constituting the gelatin particles with a crosslinking agent such as glutaraldehyde.

Patent Literature 3 discloses a method for producing gelatin particles, comprising the steps of: (1) preparing an aqueous gelatin solution; and (2) introducing the aqueous solution as droplets, e.g. through an inkjet printhead, into a solvent to form dispersed particles.

### Citation List

### Patent Literature

Patent Literature 1: JP 2014-58465 A
Patent Literature 2: JP 2008-510688 A
Patent Literature 3: WO2008/062908

### Summary of Invention

### Technical Problem

The gelatin particles disclosed in Patent Literature 1 and Patent Literature 2 are considered to be suitable for use in so-called drug delivery system (DDS) in which the gelatin particles are administered into a blood vessel, an organ, or the like to delivery and release a reagent or the like.

Meanwhile, in recent years, there is an increasing demand for the technique of directly introducing a reagent or the like into living cells. For example, when a contrast medium is introduced into living cells, the activity of the cells can be non-destructively examined. Further, when cells having a contrast medium introduced thereinto are transplanted into a patient, it is possible to externally and less-invasively examine whether or not the transplanted cells have been colonized without incising a transplantation site again. Since gelatin has high biocompatibility, gelatin particles are considered to be suitable also as carriers to carry a reagent or the like to be introduced into living cells.

An electroporation method or a microinjection method may be used to introduce gelatin particles carrying a reagent or the like into cells. However, such a method is performed by changing the shape of the cell membrane to introduce a reagent or the like into the inside of the cell membrane, and therefore there is a fear that the cell membrane is partially broken so that the activity of cells is reduced. From the viewpoint of minimizing such a reduction in the activity of cells, a reagent or the like is preferably taken up into cells by cell's own action. Therefore, gelatin particles carrying a reagent or the like are also preferably easily taken up into cells by cell's own action. However, according to the findings of the present inventors, the gelatin particles disclosed in Patent Literature 1 are hard to be taken up into cells by cell's own action.

Meanwhile, the above-described gelatin particles are required to be degraded and dissolved over a relatively long time in cells to sustainably release a carried reagent or the like in the cells. As described in Patent Literature 1 and Patent Literature 2, the time required for degradation and dissolution of gelatin particles in the living body can be controlled by crosslinking gelatin constituting the gelatin particles.

However, the crosslinking agent used in Patent Literature 2 to crosslink gelatin is often toxic to cells. Therefore, when the crosslinked gelatin particles disclosed in Patent Literature 2 are introduced into cells, there is a fear that the activity of the cells is reduced by the crosslinking agent released due to the dissolution of the gelatin particles.

In light of the above problems, it is an object of the present invention to provide a method of producing gelatin particles that have been crosslinked without using a crosslinking agent and are easily taken up into cells by cell's own action, a cell having such gelatin particles, and a method for producing a cell having such gelatin particles.

### Solution to Problem

The present invention relates to a method for producing gelatin particles, and method for producing a cell.

According to a first aspect of the present invention, there is provided a method for producing self-cross-linked gelatin particles having a particle diameter of 0.010 µm or more but 5.0 µm or less, each particle diameter defined as an average of a major-axis length and a minor-axis length of the particle, including the steps of: discharging droplets of a liquid containing melted gelatin into an atmosphere in a heating tube or a drying chamber whose difference in temperature from the droplets is 235°C or less and drying the droplets to form the gelatin into particles; and self-crosslinking the gelatin forming the particles, wherein the droplets are discharged from a nozzle of an inkjet head.

The crosslinking of the gelatin may be self-crosslinking achieved by heating.

The heating may be performed at 60°C or higher but 250°C or lower.

According to a second aspect of the present invention, there is provided a method for producing a gelatin particle-containing cell, including adding the gelatin particles formed according to the method of the first aspect of the invention, and a cell to a liquid to allow the gelatin particles to be taken up inside a cell membrane of the cell by action of the cell.

### Advantageous Effects of Invention

According to the present invention, it is possible to provide a method of producing gelatin particles that have been crosslinked without using a crosslinking agent and are easily taken up by cells themselves, a cell having such gelatin particles, and a method for producing a cell having such gelatin particles.

### Description of Embodiments

In order to achieve the above object, the present inventors have intensively studied conditions required of gelatin particles that are easily taken up into cells by cell's own action. As a result, the present inventors have found that gelatin particles that have a particle diameter of 0.010 µm or more but 5.0 µm or less and are made of self-crosslinked gelatin are easily taken up into cells by cell's own action. This finding has led to the completion of the present invention.

### 1-1. Gelatin particles

Gelatin particles that are made of self-crosslinked gelatin and have a particle diameter of 5.0 µm or less. are easily taken up by cells, and are therefore also referred to as "easy-uptake gelatin particles" in this description. The easy-uptake gelatin particles may be single particles or aggregates of two or more gelatin particles.

It is to be noted that in this description, the particle diameter, major-axis length, and minor-axis length of the gelatin particles refer to those of the gelatin particles dried by allowing them to stand in the atmosphere at 80°C for 24 hours.

The minor-axis length and major-axis length of the easy-uptake gelatin particles may be values obtained by analyzing an image taken by a scanning electron microscope (SEM). When the gelatin particles are the above-described aggregates, the major-axis length, minor-axis length, particle diameter, and aspect ratio of the gelatin particles may be the averages of the major-axis lengths, minor-axis lengths, particle diameters, and aspect ratios of a plurality of dried gelatin particles (e.g., 20 gelatin particles) randomly selected from the aggregates, respectively.

The easy-uptake gelatin particles have a particle diameter of 0.010 µm or more but 5.0 µm or less. The gelatin particles having a particle diameter of 5.0 µm or less are easily taken up into cells by cell's own action. The reason for this is considered to be that heat easily penetrates into the gelatin particles when gelatin is self-crosslinked by heating or the like, and therefore the gelatin is hard to be denatured. If the particle diameter exceeds 5.0 µm, the thickness of gelatin is large. Therefore, when self-crosslinked by, for example, heating, the gelatin particles need to be heated for a long time to crosslink inner gelatin so that gelatin located near the surfaces of the particles is denatured. Such gelatin particles whose gelatin located near the surfaces thereof has been denatured are likely to be recognized as foreign matter by cells, and are therefore hard to be taken up into cells by cell's own action. From the above viewpoint, the particle diameter of the easy-uptake gelatin particles is preferably 2.0 µm or less, more preferably 1.8 µm or less. On the other hand, the gelatin particles having a particle diameter of 0.010 µm or more can easily carry a reagent or the like therein and can easily be self-crosslinked. From the above viewpoint, the particle diameter of the gelatin particles is preferably 0.010 µm or more, more preferably 0.020 µm or more. Further, when having a particle diameter of 0.50 µm or more, the gelatin particles are excellent in handleability and can contain a large amount of reagent or the like. It is to be noted that the particle diameter of the easy-uptake gelatin particles may be the average of the major-axis length and the minor-axis length of the gelatin particles.

The easy-uptake gelatin particles preferably have an aspect ratio of 1.0 or more but 1.4 or less. When the aspect ratio is 1.4 or less, the gelatin particles are more likely to keep their nearly-spherical shape even after swelling in water. Therefore, in a solution containing the gelatin particles and cells, the gelatin particles are likely to come into contact with the cells at contact surfaces having more uniform shape and size. From this, it is considered that there is little difference in ease of uptake among the gelatin particles. Therefore, it is considered that when the easy-uptake gelatin particles have an aspect ratio within the above range, the amount of the gelatin particles to be taken up into cells and the amount of cells that take up the gelatin particles can be more easily controlled. The aspect ratio of the easy-uptake gelatin particles may be a value determined by dividing the major-axis length of the gelatin particles by the minor-axis length of the gelatin particles.

The easy-uptake gelatin particles are mainly made of gelatin. More specifically, the easy-uptake gelatin particles contain 300 or more glycine residues out of 1000 amino acid residues and contain both alanine and proline when analyzed with an amino acid analyzer. The gelatin constituting the easy-uptake gelatin particles is not particularly limited as long as it is capable of forming particles, and any known gelatin may be used which is obtained by denaturing collagen derived from cattle bone, cattle skin, pig skin, pig tendon, fish scales, and fish meat. Gelatin has previously been used for food and for medical purposes, and its intake into the body is hardly harmful to the human body. Further, gelatin disperses and disappears in the living body, and is therefore advantageous in that its removal from the living body is not required. It is to be noted that the easy-uptake gelatin particles may contain a component other than gelatin as long as the gelatin particles can be taken up into cells. It is to be noted that when the easy-uptake gelatin particles contain a component other than gelatin, the component is preferably contained to the extent that harm to the human body caused by intake into the body is negligible. Further, the component other than gelatin is preferably composed of a substance that does not accumulate in the living body and is easily discharged.

From the viewpoint of easily forming gelatin particles that satisfy the above-described conditions of particle diameter and swelling degree, the weight-average molecular weight of the gelatin constituting the easy-uptake gelatin particles is preferably 1000 or more but 100000 or less. The weight-average molecular weight may be a value measured in accordance with, for example, the PAGI Method Ver. 10 (2006).

The gelatin constituting the easy-uptake gelatin particles has been self-crosslinked. Examples of the self-crosslinking include crosslinking achieved by application of heat and crosslinking achieved by irradiation with electron beams or ultraviolet rays. In the case of the easy-uptake gelatin particles having a particle diameter of 0.010 µm or more but 5.0 µm or less, the entire gelatin inside the particles can be sufficiently crosslinked, and therefore sustained releasability can further be enhanced. Further, the gelatin can be self-crosslinked while the denaturation of the gelatin in the surfaces of the particles is reduced. Therefore, the self-crosslinked gelatin particles can be more easily taken up into cells by cell's own action.

It is to be noted that in this description, the phrase "the gelatin constituting the gelatin particles has been self-crosslinked" means that when the gelatin particles are measured with a Fourier transform infrared spectrophotometer (FT-IR) to obtain a spectrum in which wave number is plotted on the horizontal axis and absorbance is plotted on the vertical axis, the peak intensity ratio between COOH and CONH (peak intensity of COOH/peak intensity of CONH) in the spectrum is 0.46 or more. The peak intensity ratio of non-crosslinked gelatin particles is in the range of 0.40 or more but 0.44 or less. On the other hand, when gelatin is self-crosslinked, a large amount of COOH is generated, and therefore the peak intensity ratio is increased to 0.46 or more.

The reason why the peak intensity ratio between COOH and CONH is increased by self-crosslinking of gelatin is considered as follows.

Self-crosslinking of gelatin proceeds by the following two reactions.

Reaction (1): -COOH +-NH₂ → -CONH + H₂O

Reaction (2): Generation of -COOH by oxidation of constituent amino acid side chains

In the reaction (1), since the COOH group is consumed and the CONH group is generated, the peak intensity ratio is reduced. However, the reaction rate of the reaction (2) is higher than that of the reaction (1), and therefore the generation of -COOH exceeds the consumption of -COOH so that the peak intensity ratio is increased.

When the peak intensity ratio between COOH and CONH is higher, the degree of crosslinking is higher. It is considered that when the degree of crosslinking is higher, the easy-uptake gelatin particles are more slowly enzymatically degraded in cells so that a substance present in the particles is more sustainably released. However, it is considered that if the amount of COOH present on the surfaces of the easy-uptake gelatin particles is too large, the affinity between the easy-uptake gelatin particles and cells is reduced so that the efficiency of uptake of the easy-uptake gelatin particles by cells is reduced. Therefore, in view of both the efficiency of uptake of the easy-uptake gelatin particles by cells and the sustained releasability of the easy-uptake gelatin particles, the peak intensity ratio between COOH and CONH in a spectrum obtained by measuring the easy-uptake gelatin particles with a FT-IR is preferably 0.46 or more but 1.0 or less. It is considered that when the peak intensity ratio between COOH and CONH is within the above range, the efficiency of uptake of the easy-uptake gelatin particles by cells can be improved, and the easy-uptake gelatin particles can exhibit excellent sustained releasability in cells. The peak intensity ratio is more preferably 0.46 or more but 0.50 or less.

On the other hand, from the viewpoint of further reducing toxicity to living cells, the easy-uptake gelatin particles preferably have a low content of a component having a low molecular weight and derived from a crosslinking agent such as glutaraldehyde, a water-soluble carbodiimide such as 1-ethyl-3-(3-dimethylaminopropyl)carbodiimide hydrochloride or 1-cyclohexyl-3-(2-morpholinoethyl)carbodiimide-metho-p-toluenesulfonate, a bis-epoxy compound, or formalin.

The easy-uptake gelatin particles may carry a reagent or the like. The phrase "gelatin particles carry a reagent or the like" means that a reagent or the like is present on the surfaces of the gelatin particles or inside the gelatin particles. From the viewpoint of retaining the reagent or the like in cells for a longer period of time, the reagent or the like is preferably present inside the easy-uptake gelatin particles.

Examples of the reagent or the like include: reagents to be used for tests of biological activity, measurements of substances in the living body, and quantitative analysis of substances in the living body; and drugs. Examples of the reagents include contrast media.

Examples of the contrast media include magnetic substances for use as contrast media for MRI. Examples of the contrast media for MRI include contrast media containing gadolinium (Gd) and contrast media containing iron (e.g., Fe₃O₄ and γ-Fe₂O₃).

The drugs are not particularly limited as long as they can be carried by the gelatin particles. Examples of such drugs include proteins having pharmaceutical activity, plasmids, aptamers, antisense nucleic acids, ribozymes, nucleic acids used for pharmaceutical purposes, including tRNA, snRNA, siRNA, shRNA, ncRNA, and condensed DNA, and antigens used for pharmaceutical purposes.

Examples of the proteins having pharmaceutical activity include steroids, nonsteroidal anti-inflammatory drugs (NSAIDs), vitamin A (retinoid), vitamin D3 and vitamin D3 analogs, antibiotics, antiviral drugs, and antibacterial drugs.

### 1-2. Method for Producing Gelatin Particles

The easy-uptake gelatin particles can be produced by a known method in which gelatin is formed into particles and then self-crosslinked.

Examples of a method for forming gelatin into particles include: a method in which droplets of a liquid containing melted gelatin (hereinafter, also simply referred to as "gelatin solution") are discharged into an atmosphere in a heating tube or a drying chamber and dried (in-air dropping method); a method in which droplets of a gelatin solution are discharged into a hydrophobic solvent and dispersed (in-liquid dropping method); and a method in which a gelatin solution is emulsified to disperse microdroplets containing gelatin (in-liquid dispersion method). Examples of the in-air dropping method include an inkjet method and a spray drying method. Examples of the in-liquid dispersion method include an emulsion method and a coacervation method. From the viewpoint of producing gelatin particles having a more uniform particle diameter and a smaller aspect ratio, an in-air dropping method is preferred, and an inkjet method is more preferred. The method of the present invention for producing self-crosslinked gelatin particles is defined in the claims.

According to new findings by the present inventors, an in-air dropping method is particularly preferred in which the above-described droplets are dried under conditions where a temperature change is small. It is considered that this makes it possible to prevent gelatin particles from being deformed or disintegrated due to temperature change so that gelatin particles having a particle diameter of 0.010 µm or more but 5.0 µm or less are more likely to be produced and the produced gelatin particles can have a more uniform particle diameter.

For example, in the case of an inkjet method, droplets of a gelatin solution can be discharged from an inkjet nozzle provided inside a heated heating tube and collected by a filter provided inside the same heating tube. From the viewpoint of further reducing deformation or disintegration of gelatin particles, the heating of the heating tube is preferably performed by allowing hot air to flow through the inside of the heating tube in the same direction as a direction in which the droplets are dropped (i.e., in a vertical direction from the top toward the bottom of the heating tube).

Alternatively, but not according to the claimed invention, a spray drying method may be used. In this case, a gelatin solution is sprayed from an atomizer or a nozzle into a heated drying chamber.

From the viewpoint of further enhancing the above-described effects, the difference between the temperature of an atmosphere in the heating tube or the drying chamber and the temperature of a dropped gelatin solution is preferably 235°C or less. Further, from the viewpoint of efficiently and stably obtaining gelatin particles, the temperature difference is preferably 20°C or more but 200°C or less, more preferably 20°C or more but 100°C or less. Particularly, the temperature of an atmosphere in the heating tube or the drying chamber is preferably higher than the temperature of droplets by 20°C or more but 80°C or less. The temperature of droplets is preferably 15°C or higher but 80°C or lower, more preferably 20°C or higher but 50°C or lower. The temperature of an atmosphere in the heating tube or the drying chamber is preferably 40°C or higher but 250°C or lower, more preferably 40°C or higher but 150°C or lower.

From the viewpoint of making gelatin particles having a particle diameter of 0.010 µm or more but 5.0 µm or less more likely to be produced, the gelatin content of the gelatin solution is preferably 1.00 × 10⁻⁸ vol% or more but 60 vol% or less, more preferably 1.00 × 10⁻⁷ vol% or more but 50 vol% or less, even more preferably 1.00 × 10⁻⁷ vol% or more but 20 vol% or less.

Examples of a method for self-crosslinking gelatin include a method in which gelatin particles are heated and a method in which gelatin particles are irradiated with electron beams or ultraviolet rays. In the case of irradiation with electron beams or ultraviolet rays, both crosslinking and degradation reactions of gelatin occur, and which of the reactions preferentially occurs greatly depends on irradiation conditions such as atmosphere and temperature, which makes it difficult to control the degree of crosslinking. Therefore, from the viewpoint of producing gelatin particles having a crosslinking degree such that the above-described peak intensity ratio between COOH and CONH is 0.46 or more but 1.0 or less, crosslinking is preferably achieved by heating because the degree of crosslinking is relatively easily controlled.

An example of a method in which gelatin is self-crosslinked by heating gelatin particles includes a method in which gelatin particles having a particle diameter of 0.010 µm or more but 5.0 µm or less are heated at 60°C or higher but 250°C or lower, preferably 100°C or higher but 200°C or lower for 0.5 hours or longer but 100 hours or shorter.

An example of a method in which gelatin is self-crosslinked by irradiating gelatin particles with electron beams includes a method in which gelatin particles having a particle diameter of 0.010 µm or more but 5.0 µm or less are irradiated with electron beams at an acceleration voltage of 100 kV or more but 3 MV or less and an irradiation dose of 2 kGy or more but 3000 kGy or less.

An example of a method in which gelatin is self-crosslinked by irradiating gelatin particles with ultraviolet rays includes a method in which gelatin particles having a particle diameter of 0.010 µm or more but 5.0 µm or less are irradiated with ultraviolet rays with a wavelength of 250 nm or more but 260 nm or less at an integrated illumination intensity of 1 J/cm² or more but 6 J/cm² or less.

### 2. Cell

### 2-1. Cell

The cell produced according to the invention is a cell containing easy-uptake gelatin particles inside the cell membrane (hereinafter, also referred to as "gelatin particle-containing cell").

The phrase "containing gelatin particles inside the cell membrane" means that in an image of a cell taken by a TEM, gelatin particles are observed inside the cell membrane. The uptake of gelatin particles into cells can be confirmed in the following manner. For example, when gelatin particles contain a contrast medium, the determination as to whether or not the gelatin particles containing a contrast medium have been taken up into cells can be made by staining and microscopically observing the contrast medium. When not containing a contrast medium, gelatin particles may be previously fluorescently labeled. In this case, the determination as to whether or not the fluorescently labeled-gelatin particles have been taken up into cells can be made using a confocal microscope. The fluorescent labeling of gelatin particles can be performed using, as a substrate, FITC-gelatin prepared by, for example, mixing equal amounts of a solution labeled with fluorescein isothiocyanate (FITC) (e.g., a 10 mM acetic acid solution of FITC-collagen manufactured by Cosmo Bio Co., Ltd.), 0.4 M sodium chloride, 0.04% (W/V) sodium azide, and a 50 mM tris-HCl buffer containing 10 mM calcium chloride (pH 7.5) and then heating the mixture at 60°C for 30 minutes.

The easy-uptake gelatin particles contained in cells preferably carry a contrast medium, especially a contrast medium for MRI. Such cells are produced by a method described later in which easy-uptake gelatin particles are taken up into cells by cell's own action. Therefore, the activity of the cells can be non-destructively examined by observing the presence or absence of the contrast medium in the cells.

Examples of cells capable of containing gelatin particles inside the cell membrane include known cells including cells derived from biological samples or specimens extracted from various organs such as bone marrow, heart, lung, liver, kidney, pancreas, spleen, intestinal tract, small intestine, cardiac valve, skin, blood vessel, cornea, eyeball, dura mater, bone, trachea, and auditory ossicles, commercially-available established cell lines, stem cells such as skin stem cells, epidermal keratinocyte stem cells, retinal stem cells, retinal epithelial stem cells, cartilage stem cells, hair follicle stem cells, muscle stem cells, osteoprogenitor stem cells, preadipocyte stem cells, hematopoietic stem cells, nerve stem cells, hepatic stem cells, pancreatic stem cells, ectodermal stem cells, mesodermal stem cells, endodermal stem cells, mesenchymal stem cells, ES cells, and iPS cells, and cells differentiated from these stem cells.

Among these cells, when cells, especially stem cells or cells differentiated from stem cells, to be transplanted into a patient in regenerative medicine contain easy-uptake gelatin particles carrying a contrast medium, especially a contrast medium for MRI, the determination as to whether or not the gelatin particle-containing cells have been colonized in a transplantation site can be made without reoperation by observing the contrast medium in the transplantation site after transplantation into a patient. Therefore, it is considered that these cells containing gelatin particles carrying a contrast medium for MRI can reduce the physical, mental, financial, and temporal burden on patients who receive regenerative medicine treatment and improve the quality of life (QOL) of the patients.

### 2-2. Method for producing cell

The gelatin particle-containing cell can be produced by introducing easy-uptake gelatin particles into the cell mentioned above. Examples of a method for introducing gelatin particles into a cell include a method in which gelatin particles and a cell are added to a liquid to allow the gelatin particles to be taken up into the cell by cell's own action such as endocytosis and a method in which gelatin particles are introduced into a cell by external operation. Examples of the method in which gelatin particles are taken up into a cell by cell's own action include a method in which gelatin particles and cells are stirred in a liquid and a method in which cells are cultured in a cell culture medium containing gelatin particles. It is to be noted that the above-described easy-uptake gelatin particles are very efficiently taken up by cells themselves, and therefore it is not particularly necessary to perform operation for forming a complex with another component to promote uptake into cells. From the viewpoint of minimizing a reduction in the activity of cells, among the above methods, a method is preferred in which cells are mixed with easy-uptake gelatin particles in a liquid and cultured. Examples of the method in which gelatin particles are introduced into a cell by external operation include an electroporation method and a microinjection method. Among them, from the viewpoint of preventing a reduction in the activity of cells during the introduction of gelatin particles, a method in which gelatin particles are introduced into a cell by cell's own action is preferred, and a method in which gelatin particles are taken up into a cell without forming the above-described complex is more preferred. The method of the present invention for producing a gelatin particle-containing cell is defined in the claims.

The liquid to which gelatin particles and cells are added may be a cell culture medium. Examples of the cell culture medium include a Hanks culture medium and a HEPES culture medium. The cell culture medium may be a known buffer or physiological saline, and examples thereof include Hanks' Balanced Salt Solution (HBSS), 4-(2-hydroxyethyl)-1-piperazineethanesulfonic acid (HEPES), and another known phosphate buffered saline (PBS).

From the viewpoint of enhancing the activity of cells to facilitate the uptake of gelatin particles into the cells by cell's own action, the temperature of the cell culture medium during stirring is preferably 15°C or higher but 50°C or lower, more preferably 35°C or higher but 45°C or lower.

When gelatin particles are introduced into the inside of the cell membrane by cell's own action, introduction of gelatin particles may be promoted by, for example, shaking the cell culture medium containing the gelatin particles and the cells.

It is considered that when gelatin particles are taken up inside the cell membrane by cell's own action, high-activity cells are more likely to take up gelatin particles, but low-activity cells are less likely to take up gelatin particles. Therefore, the activity of cells can be non-destructively examined by adding gelatin particles carrying a contrast medium and cells to a liquid and, if necessary, shaking the liquid, and then by observing the presence or absence of the contrast medium in the cells.

### Examples

Hereinbelow, specific examples of the present invention will be described. It is to be noted that the scope of the present invention should not be construed as being limited to these examples.

### 1. Production of Gelatin Particles

### 1-1. Preparation of Raw Material Solutions

Gelatin (G-2613P manufactured by Nitta Gelatin Inc.), pure water, Fe₂O₃ powder (3310DX (α-Fe₂O₃) manufactured by COREFRONT Corporation) were mixed to prepare a raw material solution containing the gelatin and the Fe₂O₃ powder in a volume ratio of 10 : 1 (gelatin : Fe₂O₃ powder = 10 : 1). The amounts of the gelatin and the Fe₂O₃ powder were adjusted so that the raw material solution had a gelatin concentration and a Fe₂O₃ concentration shown in Table 1. In this way, a plurality of raw material solutions different in the ratio of solid to pure water were obtained.

### 1-2. Production of Gelatin Particles by Inkjet Method

An air flow of 3L/min was blown into a heating tube heated to 100°C in a vertical direction from the top toward the bottom of the heating tube. Droplets of 4 pL or 42 pL of each of the raw material solutions heated to 40°C were dropped from an inkjet head (512S manufactured by Konica Minolta Co., Ltd.) into the air flow at an ejecting frequency of 5 kHz and landed on a hydrophilically-treated polytetrafluoroethylene resin (PTFE) filter (Millipore, 0.45 mesh, manufactured by Merck (Nihon Millipore K.K.)) located 200 cm below the inkjet head. The dropping was performed for 5 hours, and then gelatin particles on the filter were collected.

### 1-3. Production of Gelatin Particles by Spray Drying Method (not according to the invention)

A predetermined one of the above-described raw material solutions was sprayed from a two-fluid-type nozzle of a spray dryer (SPRAY BOY manufactured by PRECI Co., Ltd.) into a drying chamber heated to 200°C at a rate of 1 kg/h, and gelatin particles were collected from the bottom of the drying chamber.

### 1-4. Crosslinking

The non-crosslinked gelatin particles produced above were heated in a vacuum heating furnace (Vacuum Oven ADP200 manufactured by Yamato Scientific Co., Ltd.) at 160°C for a predetermined time shown in Table 1. In this way, self-crosslinked Gelatin Particles 1 to 9 and self-crosslinked Gelatin Particles 12 to 18 were obtained.

Further, the non-crosslinked gelatin particles produced above using a predetermined one of the raw material solutions were put into an emulsion obtained by dispersing glutaraldehyde in isopropanol, and the mixture was stirred for 12 hours to crosslink the gelatin particles with glutaraldehyde. In this way, crosslinked Gelatin Particle 10 and crosslinked Gelatin Particle 11 were obtained.

Table 1 shows the volume percentages of gelatin and Fe₂O₃ in each of the raw material solutions used for producing Gelatin Particles 1 to 18, a particle production method, the quantity of each droplet when particles were produced by an inkjet method, a crosslinking method, heating time for self-crosslinking, and the concentration of the crosslinking agent added. It is to be noted that in the column "Crosslinking Method" in Table 1, "self-crosslinking" means that gelatin particles were self-crosslinked by heating, and "crosslinking agent" means that gelatin particles were crosslinked with glutaraldehyde. The embodiments using the crosslinking agent are not according to the invention.

### [Table 1]

**Table 1: Production of Gelatin Particles 1 to 18**

| | Raw material solution | | Particle production | | Crosslinking | | |
|---|---|---|---|---|---|---|---|
| | Gelatin (vol%) | Fe₂O₃ (vol%) | Method | Quantity of droplet (pL) | Crosslinking method | Heating time (hr) | Concentration of crosslinking agent (mg/L) |
| Gelatin Particle 1 | 4.88 × 10⁻⁷ | 4.88 × 10⁻⁸ | Inkjet | 4 | Self-crosslinking | 48 | - |
| Gelatin Particle 2 | 6.10 × 10⁻² | 6.10 × 10⁻³ | Inkjet | 4 | Self-crosslinking | 48 | - |
| Gelatin Particle 3 | 0.488 | 4.88 × 10⁻² | Inkj et | 4 | Self-crosslinking | 48 | - |
| Gelatin Particle 4 | 1.65 | 0.165 | Inkjet | 4 | Self-crosslinking | 48 | - |
| Gelatin Particle 5 | 4.88 × 10⁻⁷ | 4.88 × 10⁻⁸ | Inkjet | 4 | Self-crosslinking | 10 | - |
| Gelatin Particle 6 | 6.10 × 10⁻² | 6.10 × 10⁻³ | Inkjet | 4 | Self-crosslinking | 10 | - |
| Gelatin Particle 7 | 0.488 | 4.88 × 10⁻² | Inkjet | 4 | Self-crosslinking | 30 | - |
| Gelatin Particle 8 | 1.65 | 0.165 | Inkjet | 4 | Self-crosslinking | 10 | - |
| Gelatin Particle 9 | 6.10 × 10⁻² | 6.10 × 10⁻³ | Spray drying | - | Self-crosslinking | 10 | - |
| Gelatin Particle 10 | 6.10 × 10⁻² | 6.10 × 10⁻³ | Inkjet | 4 | Crosslinking agent | - | 0.1 |
| Gelatin Particle 11 | 7.75 × 10⁻⁴ | 7.75 × 10⁻⁵ | Inkjet | 4 | Crosslinking agent | - | 0.1 |
| Gelatin Particle 12 | 46.5 | 4.65 | Inkjet | 42 | Self-crosslinking | 10 | - |
| Gelatin Particle 13 | 6.10 × 10⁻² | 6.10 × 10⁻³ | Inkjet | 4 | Self-crosslinking | 10 | - |
| Gelatin Particle 14 | 6.10 × 10⁻² | 6.10 × 10⁻³ | Inkjet | 4 | Self-crosslinking | 10 | - |
| Gelatin Particle 15 | 6.10 × 10⁻² | 6.10 × 10⁻³ | Inkjet | 4 | Self-crosslinking | 30 | - |
| Gelatin Particle 16 | 6.10 × 10⁻² | 6.10 × 10⁻³ | Inkjet | 4 | Self-crosslinking | 48 | - |
| Gelatin Particle 17 | 6.10 × 10⁻² | 6.10 × 10⁻³ | Inkjet | 4 | Self-crosslinking | 70 | - |
| Gelatin Particle 18 | 6.10 × 10⁻² | 6.10 × 10⁻³ | Inkjet | 4 | Self-crosslinking | 90 | - |

### 2. Measurement of Gelatin Particles

### 2-1. Average Particle Diameter and Aspect Ratio

Gelatin particles of each of Gelatin Particles 1 to 18 produced above were imaged with a scanning electron microscope (SEM). The taken image was analyzed using Mac-View that is particle size distribution analysis software manufactured by Mountech Co., Ltd. to measure the minor-axis lengths and the major-axis lengths of randomly-selected 20 gelatin particles, and the averages thereof were defined as the minor-axis length and the major-axis length of each of Gelatin Particles 1 to 18. The average of the minor-axis length and the major-axis length of the gelatin particles was defined as the average particle diameter of each of Gelatin Particles 1 to 18. Further, the aspect ratios of randomly-selected 10 gelatin particles were measured, and the average thereof was defined as the aspect ratio of each of Gelatin Particles 1 to 18.

Table 2 shows the average particle diameter and the aspect ratio of each of Gelatin Particles 1 to 18. The average particle diameter of Gelatin Particle 12 is not according to the invention.

### [Table 2]

**Table 2: Average Particle Diameters and Aspect Ratios of Gelatin Particles 1 to 18**

| | Average particle diameter (µm) | Aspect ratio |
|---|---|---|
| Gelatin Particle 1 | 0.03 | 1.05 |
| Gelatin Particle 2 | 1.5 | 1.05 |
| Gelatin Particle 3 | 3.0 | 1.05 |
| Gelatin Particle 4 | 4.5 | 1.05 |
| Gelatin Particle 5 | 0.03 | 1.3 |
| Gelatin Particle 6 | 1.5 | 1.1 |
| Gelatin Particle 7 | 3.0 | 1.1 |
| Gelatin Particle 8 | 4.5 | 1.1 |
| Gelatin Particle 9 | 1.5 | 2.0 |
| Gelatin Particle 10 | 1.5 | 1.3 |
| Gelatin Particle 11 | 0.5 | 1.3 |
| Gelatin Particle 12 | 30 | 1.1 |
| Gelatin Particle 13 | 1.5 | 1.1 |
| Gelatin Particle 14 | 1.5 | 1.1 |
| Gelatin Particle 15 | 1.5 | 1.07 |
| Gelatin Particle 16 | 1.5 | 1.05 |
| Gelatin Particle 17 | 1.5 | 1.05 |
| Gelatin Particle 18 | 1.5 | 1.03 |

### 2-2. Peak Intensity Ratio between COOH and CONH

Each of Gelatin Particles 13 to 18 produced above was measured with a Fourier transform infrared spectrophotometer (FT-IR) to obtain a spectrum in which wave number was plotted on the horizontal axis and absorbance was plotted on the vertical axis. The measurement conditions were as follows.
Measuring apparatus: Fourier transform infrared spectrophotometer, Nicolet 380 (manufactured by Thermo Fisher Scientific)
FT-IR measurement conditions: Single reflection ATR
Detection range: 4000-700 cm-1
Number of scans: 32 times
Window material: Ge
Resolution: 4
Beam splitter: KBr
Gain: 2
Light source: IR
Detector: DTGS KBr

The ratio between the peak intensity of COOH and the peak intensity of CONH in the obtained spectrum was determined and defined as a peak intensity ratio between COOH and CONH (COOH/CONH).

Table 4 shows COOH/CONH of each of Gelatin Particles 13 to 18.

### 3. Introduction into Cells and Evaluations

### 3-1. Introduction into Cells

A cell culture medium was used which was prepared by adding 50 mL of fetal bovine serum to 500 mL of a cell culture medium MEM Alpha basic (1X) manufacture by Life Technologies. One milligram of each of Gelatin Particles 1 to 18 was added to 3 mL of the cell culture medium, and mouse osteoblast-derived cells (MC3T3E1) were added at 6000 cells/mL. The cell culture medium after cell addition was incubated at 40°C for 24 hours. In this way, 18 evaluation samples were prepared.

For each of Gelatin Particles 1 to 12, another evaluation sample was prepared by extending the incubation time after cell addition to 48 hours. In this way, 12 evaluation samples were prepared.

### 3-2. Evaluation of Uptake by Cells

Part of the cell suspension was taken from each of the evaluation samples and observed in the following manner to determine whether or not gelatin taken up inside the cell membrane could be confirmed. The uptake of gelatin particles by cells was evaluated according to the following criteria.

### (Staining of Cells and Fe)

First, 1 ml of 1% paraformaldehyde was added to the cultured cells to perform cell immobilization treatment. Then, 1 ml of an Fe staining solution having the following composition was added to stain Fe. Further, 1 mL of a nuclear staining solution adjusted to the following concentration was added to stain the cells.

### (Composition of Fe Staining Solution)

Equal volumes of the following two solutions were mixed to prepare an Fe staining solution.
- 20 vol% HCL (5-fold dilution of concentrated hydrochloric acid)
- 10 mass% aqueous K₄(Fe(CN₆)) solution (100 mg/mL)

### (Composition of Nuclear Staining Solution)

Five parts by mass of ammonium sulfate and 0.1 parts by mass of Nuclear fast red were mixed with 100 parts by mass of distilled water to prepare a nuclear staining solution.

### (Counting of Number of Cells That Have Taken up Fe)

The stained cells were observed with an optical microscope to evaluate whether blue-stained Fe was contained in randomly-selected 20 cells.
⊙ Out of the above 20 cells, 50% or more of the cells (10 or more cells) were confirmed to have taken up gelatin inside the cell membrane.
∘ Out of the above 20 cells, 10% or more but less than 50% of the cells (2 or more but less than 10 cells) were confirmed to have taken up gelatin inside the cell membrane.
Δ Out of the above 20 cells, less than 10% of the cells (less than 2 cells) were confirmed to have taken up gelatin inside the cell membrane.
× Out of the above 20 cells, no cells were confirmed to have taken up gelatin.

### 3-3. Evaluation of cell viability

The viability of cells that had taken up each of Gelatin Particles 1 to 12 was evaluated using each of the evaluation samples of Gelatin Particles 1 to 12.

Each of the evaluation samples was maintained at 40°C. After 1 week, the cell culture medium was changed to a new one once, and the cells were cultured for 2 weeks. Then, 20 cells were randomly selected from cells in the culture medium after cultivation and observed using Live/Dead Cell Staining Kit II manufactured by Takara Bio Inc. to determine whether the 20 cells were alive or dead. The viability of the cells was evaluated according to the following criteria.
∘ Out of the 20 cells, 70% or more of the cells (14 or more cells) were alive.
× Out of the above 20 cells, less than 70% of the cells (less than 14 cells) were alive.

### 3-4. Enzymatic Degradation Time (Degradation Test)

The time required to degrade the gelatin particles by an enzyme contained in cells was measured in the following manner using each of the evaluation samples of Gelatin Particles 13 to 18.

One milliliter of the cell suspension was added to each well of 12-well multiwell plates (Coming) so that 5 × 10 cells were inoculated into each well. It is to be noted that the number of wells into which cells were inoculated was 17 per each of Gelatin Particles 13 to 18.

After the cells were incubated at 40°C for 24 hours, the culture medium was removed, and 1 mL of a culture medium containing the gelatin particles (concentration of gelatin particles: 200 µg/ml) was added to each well. The cells were further incubated at 40°C for 24 hours, and then the culture medium was removed and the cells were washed with PBS (Gibco) three times.

Then, 1 mL of a 0.25 mass% trypsin-EDTA solution (0.25% trypsin-EDTA (1×) containing phenol red) (Gibco) was added to the cells in each well to detach the cells from the well. The detached cells were then again inoculated into new 12-well multiwell plates and incubated at 40°C. The concentration of Fe in the cells was measured in the following manner after 0, 1, 3, 5, 7, 10, 12, 14, 16, 18, 20, 25, 30, 35, 40, 50, and 60 days from the start of the incubation.

### (Measurement of Fe Concentration in Cells)

The culture medium was removed from the well in which the cells were incubated, and the cells were washed with PBS three times. Then, a 1 M hydrochloric acid solution (manufactured by Wako Pure Chemical Industries, Ltd.) was added to the cells, and the mixture was allowed to stand for 5 minutes to dissolve the cells. The cell lysate in the well was collected into an Eppendorf tube, and the quantity of Fe was determined by an atomic absorption photometer A4-6800 (manufactured by Shimadzu Corporation).

### (Evaluation Method)

The quantity of Fe obtained from the cells on day 0 after the start of incubation was defined as a reference value (initial quantity of Fe), and the time required to reduce the measured quantity of Fe to 10% or less of the initial quantity of Fe was defined as enzymatic degradation time.

The evaluation results of Gelatin Particles 1 to 12 are shown in Table 3, and the evaluation results of Gelatin Particles 13 to 18 are shown in Table 4.

### [Table 3]

**Table 3: Evaluation Results of Gelatin Particles 1 to 12**

| | Uptake by cells (after 24 hours) | Uptake by cells (after 48 hours) | Cell viability |
|---|---|---|---|
| Gelatin Particle 1 | ⊙ | ⊙ | ○ |
| Gelatin Particle 2 | ⊙ | ⊙ | ○ |
| Gelatin Particle 3 | ○ | ⊙ | ○ |
| Gelatin Particle 4 | ○ | ⊙ | ○ |
| Gelatin Particle 5 | ⊙ | ⊙ | ○ |
| Gelatin Particle 6 | ⊙ | ⊙ | ○ |
| Gelatin Particle 7 | ○ | ⊙ | ○ |
| Gelatin Particle 8 | ○ | ⊙ | ○ |
| Gelatin Particle 9 | ○ | ⊙ | ○ |
| Gelatin Particle 10 | ⊙ | ⊙ | × |
| Gelatin Particle 11 | ⊙ | ⊙ | × |
| Gelatin Particle 12 | × | Δ | ○ |

Gelatin particles 1 to 9 having an average particle diameter of 0.010 µm or more but 5.0 µm or less and made of self-crosslinked gelatin were easily taken up into cells by cell's own action.

In particular, the gelatin particles having an aspect ratio of 1.0 or more but 1.4 or less were generally easily taken up into cells in a short time. For example, Gelatin Particles 2 and 6 were easily taken up into cells in a shorter time as compared with Gelatin Particle 9 having the same average particle diameter as Gelatin Particles 2 and 6 and an aspect ratio larger than 1.4. It is to be noted that the gelatin particles produced by an inkjet method generally had a small aspect ratio. In particular, Gelatin Particles 2 and 6 had a smaller aspect ratio than Gelatin Particle 9 produced by a spray drying method and having the same average particle diameter as Gelatin Particles 2 and 6.

Further, Gelatin Particles 1 and 2 having an average particle diameter of 0.010 µm or more but 2.0 µm or less were easily taken up into cells in a shorter time as compared with Gelatin Particles 3 and 4 having the same aspect ratio as Gelatin Particles 1 and 2 and an average particle diameter larger than 2.0 µm. Similarly, Gelatin Particle 6 having an average particle diameter of 0.010 µm or more but 2.0 µm or less was easily taken up into cells in a shorter time as compared with Gelatin Particles 7 and 8 having the same aspect ratio as Gelatin Particle 6 and an average particle diameter larger than 2.0 µm.

On the other hand, the viability of cells after 2 weeks from the uptake of Gelatin Particle 10 or 11 crosslinked with glutaraldehyde was low. The reason for this is considered to be that the activity of cells was reduced by glutaraldehyde toxic to cells.

Further, Gelatin Particle 12 having an average particle diameter larger than 5.0 µm was hard to be taken up into cells by cell's own action.

### [Table 4]

**Table 4: Evaluation Results of Gelatin Particles 13 to 18**

| | COOH/CONH ratio | Uptake by cells (after 24 hours) | Enzymatic degradation time |
|---|---|---|---|
| Gelatin Particle 13 | 0.46 | ⊙ | 7 days |
| Gelatin Particle 14 | 0.50 | ⊙ | 9 days |
| Gelatin Particle 15 | 0.70 | ○ | 12 days |
| Gelatin Particle 16 | 1.00 | ○ | 14 days |
| Gelatin Particle 17 | 1.50 | Δ | 40 days |
| Gelatin Particle 18 | 2.00 | Δ | 60 days |

Gelatin particles 13 to 16 having a peak intensity ratio between COOH and CONH of 0.46 or more but 1.00 or less were easily taken up into cells by cell's own action, and their enzymatic degradation time was 7 to 14 days. The reason for this is considered to be that the gelatin particles are self-crosslinked and have a moderately high crosslinking degree, and are therefore not easily enzymatically degraded.

On the other hand, Gelatin Particles 17 and 18 having a peak intensity ratio between COOH and CONH exceeding 1.00 were harder to be taken up into cells by cell's own action as compared with Gelatin Particles 13 to 16. The reason for this is considered to be that the amount of COOH present on the surfaces of the gelatin particles was increased due to an increase in crosslinking degree so that affinity between the gelatin particles and cells was reduced. The enzymatic degradation time of Gelatin Particles 17 and 18 was as long as 40 days or more, and therefore Gelatin Particles 17 and 18 exhibited higher sustained releasability. The reason for this is considered to be that Gelatin Particles 17 and 18 were hard to be enzymatically degraded due to the high crosslinking degree of gelatin.

This application is based upon and claims the benefit of priority from prior Japanese Patent Application No. 2015-254954 filed on December 25, 2015 in Japan and prior Japanese Patent application No 2016-124507 filed on June 23, 2016 in Japan.

### Industrial Applicability

The gelatin particles produced according to the present invention can contain, for example, a contrast medium for MRI and can be introduced into cells for transplantation used in regenerative medicine. The activity of such cells can be non-destructively measured by observing the MRI image of the cells to determine whether or not the gelatin particles have been taken up into the cells. Therefore, it is considered that the use of the gelatin particles produced according to the present invention makes it possible to reduce the wastage rate of cells used in regenerative medicine and increase the use efficiency of the cells. Further, when such cells are transplanted, a transplantation site can be observed not by reoperation but by MRI imaging to determine whether or not the cells have been colonized in the transplantation site. Therefore, it is considered that the gelatin particles produced according to the present invention can reduce the physical, mental, financial, and temporal burdens on patients who receive cell transplantation and improve the quality of life (QOL) of the patients.

## Claims

1. A method for producing self-crosslinked gelatin particles having particle diameters of 0.010 µm or more but 5.0 µm or less, each particle diameter defined as an average of a major-axis length and a minor-axis length of the particle, comprising the steps of: discharging droplets of a liquid containing melted gelatin into an atmosphere in a heating tube or a drying chamber whose difference in temperature from the droplets is 235°C or less and drying the droplets to form the gelatin into particles; and self- crosslinking the gelatin forming the particles, wherein the droplets are discharged from a nozzle of an inkjet head.

2. The method for producing gelatin particles according to claim 1, wherein the self-crosslinking of the gelatin is self-crosslinking achieved by heating.

3. The method for producing gelatin particles according to claim 2, wherein the heating is performed at 60°C or higher but 250°C or lower.

4. A method for producing a gelatin particle-containing cell, comprising adding the gelatin particles formed according to claim 1 and a cell to a liquid to allow the gelatin particles to be taken up inside a cell membrane of the cell by action of the cell.

## Patentansprüche

1. Verfahren zur Herstellung selbstvernetzter Gelatinepartikel mit Partikeldurchmessern von 0,010 µm oder mehr, aber 5,0 µm oder weniger, wobei jeder Teilchendurchmesser als Mittelwert einer Hauptachsenlänge und einer Nebenachsenlänge des Teilchens definiert ist, umfassend die Schritte: Ausstoßen von Tröpfchen einer geschmolzene Gelatine enthaltenden Flüssigkeit in eine Atmosphäre in einem Heizrohr oder einer Trockenkammer, deren Temperaturdifferenz zu den Tröpfchen 235°C oder weniger beträgt, und Trocknen der Tröpfchen, wobei die Gelatine Teilchen bildet; und Selbstvernetzen der die Teilchen bildenden Gelatine, wobei die Tröpfchen aus einer Düse eines Tintenstrahlkopfes ausgestoßen werden.

2. Verfahren zur Herstellung von Gelatinepartikeln nach Anspruch 1, wobei die Selbstvernetzung der Gelatine eine durch Erhitzen bewirkte Selbstvernetzung ist.

3. Verfahren zur Herstellung von Gelatinepartikeln nach Anspruch 2, wobei das Erhitzen bei 60°C oder höher, jedoch 250°C oder niedriger durchgeführt wird.

4. Verfahren zur Herstellung einer Gelatinepartikel enthaltenden Zelle, umfassend die Zugabe der gemäß Anspruch 1 gebildeten Gelatinepartikel und einer Zelle zu einer Flüssigkeit, so dass die Gelatinepartikel durch die Wirkung der Zelle in eine Zellmembran der Zelle aufgenommen werden können.

## Revendications

1. Procédé pour produire des particules de gélatine auto-réticulées ayant des granulométries supérieures ou égales à 0,010 µm mais inférieures ou égales à 5,0 µm, chaque granulométrie étant définie par la moyenne de la longueur de l'axe majeur et de la longueur de l'axe mineur de la particule, comprenant les étapes consistant à : décharger des gouttelettes d'un liquide contenant de la gélatine fondue dans une atmosphère dans un tube chauffant ou une chambre de séchage dont la différence de température avec les gouttelettes est de 235°C ou moins, et sécher les gouttelettes pour mettre la gélatine sous forme de particules ; et auto-réticuler la gélatine formant les particules, les gouttelettes étant déchargées depuis une buse d'une tête à jet d'encre.

2. Procédé pour produire des particules de gélatine selon la revendication 1, dans lequel l'auto-réticulation de la gélatine est une auto-réticulation obtenue par chauffage.

3. Procédé pour produire des particules de gélatine selon la revendication 2, dans lequel le chauffage est effectué à une température supérieure ou égale à 60°C mais inférieure ou égale à 250°C.

4. Procédé pour produire une cellule contenant des particules de gélatine, comprenant l'étape consistant à ajouter les particules de gélatine formées selon la revendication 1 et une cellule à un liquide pour permettre aux particules de gélatine d'être captées à l'intérieur d'une membrane cellulaire de la cellule par l'action de la cellule.
